# EUROPEAN PATENT SPECIFICATION

(11) **EP 3 413 988 B1**
(45) Date of publication and mention of the grant of the patent: **20.10.2021**
(21) Application number: 17749924.1
(22) Date of filing: 09.02.2017
(51) Int. Cl.: B01B 1/00, C01B 15/01, A61L 2/20, B01D 1/14, C08K 7/02

(54) **PROCESS AND DEVICE FOR GENERATING VAPOROUS HYDROGEN PEROXIDE**
VERFAHREN UND VORRICHTUNG ZUR ERZEUGUNG VON DAMPFFÖRMIGEM WASSERSTOFFPEROXID
PROCÉDÉ ET DISPOSITIF DE PRODUCTION DE PEROXYDE D'HYDROGÈNE VAPOREUX

(30) Priority: 09.02.2016 FI 20165089
(43) Date of publication of application: 19.12.2018
(73) Proprietor: Teknologian tutkimuskeskus VTT Oy, 02150 Espoo (FI)
(72) Inventor: HEINONEN, Kimmo, 02044 VTT (FI); KULMALA, Ilpo, 02044 VTT (FI); HUMPPI, Tarmo, 34110 Lakiala (FI)
(74) Representative: Laine IP Oy
(86) International application number: PCT/FI2017/050075
(87) International publication number: WO 2017/137665

(56) References cited:
- WO-A1-03/082355
- WO-A1-2009/008755
- WO-A1-2015/059336
- WO-A2-2012/059726
- FR-A1- 2 944 705
- JP-A- 2000 070 353
- US-A1- 2013 078 143

## Description

### Background of the Invention

### Field of the Invention

The present invention concerns a process for generating vaporous hydrogen peroxide, particularly by using a simple, small-scale device that does not require pressurization. The generated hydrogen peroxide can be used, for example, for decontamination.

### Description of Related Art

Certain industries, such as the food and medical industries require a high degree of microbial purity in their products. Likewise, facilities, such as hospitals, and vehicles, such as ambulances, have similar requirements. Microbial disinfection is an important part of the used contamination control procedures. The word decontamination is used here to describe the process of reducing microbial contamination to a safe level.

Decontamination can be divided into physical, chemical and biological decontamination.

Physical methods include washing, treatment by hot air or steam, natural degradation, radiation, the use of UV light and cold plasma treatment.

Chemical methods include oxidation by chlorine compounds or hydrogen peroxide, alkaline hydrolysis and ozone treatment. As an example, JPH09272515 A describes the combined use of two different sterilization methods, one using steam and the other using hydrogen peroxide.

Biological methods include the use of bacteria, synthetic bacteria or enzymes.

The use of gaseous products is preferred over other alternatives, since the gaseous products make it possible to disinfect targets and spaces of different sizes, including large areas and facilities, and are capable of providing and maintaining an even concentration of gaseous components in the entire space to be decontaminated, they can be used at room temperature, they eliminate the human factor (compared to manual purification), and they are efficient and relatively gentle.

Several different gases have been used for decontamination. A few of the most common ones are chlorine dioxide (ClO₂), aldehydes (particularly formaldehyde) and hydrogen peroxide (H₂O₂). Of these alternatives, hydrogen peroxide is, in addition to being the safest one, also the most convenient one, as chlorine dioxide corrodes metals and formaldehyde, a suspected carcinogen, leaves a layer on the decontaminated surfaces.

Vapour generating systems are described in, e.g., US 20120277662, where the system generates free radical rich effluent from a free radical electrical generator and/or a vaporizer, equipped with a closed loop circulating system. A similar system is described in US 5792435, where the generator includes one or more vaporizers that inject a combination of a carrier gas and a vaporized decontaminant into an isolation chamber. Both of these include the use of complex devices with a closed circulation of gas. JP2000070353 A discloses another device for generating vapour and using it in an air cleaner.

US2011176959 A1 also describes a method for sterilizing objects using hydrogen peroxide. Since the method requires the use of a vacuum, the objects to be sterilized must be of a limited size. Similarly, WO9715333 A1 describes a process for sterilizing medical instruments using hydrogen peroxide vapour. WO2015/059336 A1 discloses another process for producing hydrogen peroxide and using it for purifying air.

US20140079597 describes a device for vaporizing a liquid from a wick. However, the surface area available for evaporation is small (evaporation occurs only from the end of the wick), and the formed vapour can travel out from the vaporization vessel, along the wick, only through a small outlet in a lid, through which the wick has been guided, whereby the rate of vapour generation remains low.

Although it is known that hydrogen peroxide vapour can be used for decontamination or sterilization purposes in a relatively safe and efficient method, there is still a need for a safe, reliable and robust method of generating the required hydrogen peroxide vapour at sufficiently high rates.

Currently it is typically done by evaporating H₂O₂ from liquid solutions. However, pressurized systems have been used for this purpose (commonly, a pressure of 4 bars is required), to ensure safe boiling of hydrogen peroxide.

### Summary of the Invention

The present invention is defined by the features of the independent claims. Some specific embodiments are defined in the dependent claims.

According to a first aspect of the present invention, there is provided a device suitable for generating vaporous hydrogen peroxide (H₂O₂).

According to a second aspect of the present invention, there is provided a process for generating vaporous hydrogen peroxide (H₂O₂).

According to a third aspect of the present invention, there is provided a use of said device or said process in hospitals, in the food industry, or in transport vehicles or pods, or defense or security sectors.

The general idea of the invention is to vaporize hydrogen peroxide into the air from a container using a capillary conveyer and a temperature controlled carrier gas supply. The capillary conveyer consists of a large number of thin non-absorbing solid fibres (e.g glass fibres) forming totally a large area for evaporation. Typically, a fabric system is comprised of many tows, each of which consists of numerous individual fibers; therefore, there are capillary gaps between fibers in a tow, as well as larger gaps between tows in a fabric.

Using this procedure, large amounts of vaporized hydrogen peroxide can be generated in a safe and simple way.

An efficient process requires control of the temperature and humidity. This might require drying, which on the other hand can be easily done using e.g. a condensing or absorbing dryer. Further, an even concentration should be obtained and maintained in the entire area to be decontaminated. After the decontamination, the area should be ventilated and the ventilated air should be filtered.

The invention solves the main shortcomings of current generation methods, namely the need for complex and expensive equipment for hydrogen peroxide vapor generation. The device of the present invention is therefore simple, robust, affordable and safe to use. Moreover, it does not need pressurization. Without pressurization, heating hydrogen peroxide can cause it to dissolve violently which is a potential risk. Thus, the equipment needed for decontamination can be reduced to minimum.

The use of hydrogen peroxide vapor for decontamination, in general, has the advantage of not causing corrosion, whereas hydrogen peroxide in solution would.

### Brief Description of the Drawings

FIGURE 1 is a schematic drawing of the device of an embodiment of the invention.
FIGURE 2 is a photograph of an exemplary device of an embodiment of the invention.
FIGURE 3 is a graph illustrating the achieved concentration of hydrogen peroxide when using the process of an embodiment of the invention for disinfecting a room of 33 m³.

### Embodiments of the Invention

It should be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

Furthermore, the described features, structures, or characteristics may be combined in any suitable manner in one or more embodiments. In this description, specific details are provided, such as examples of lengths, widths, etc., to provide a thorough understanding of embodiments of the invention. One skilled in the relevant art will recognize, however, that the invention can be practiced without one or more of the specific details, or with other similar details.

Accordingly, it is not intended that the invention be limited, except as by the claims set forth below.

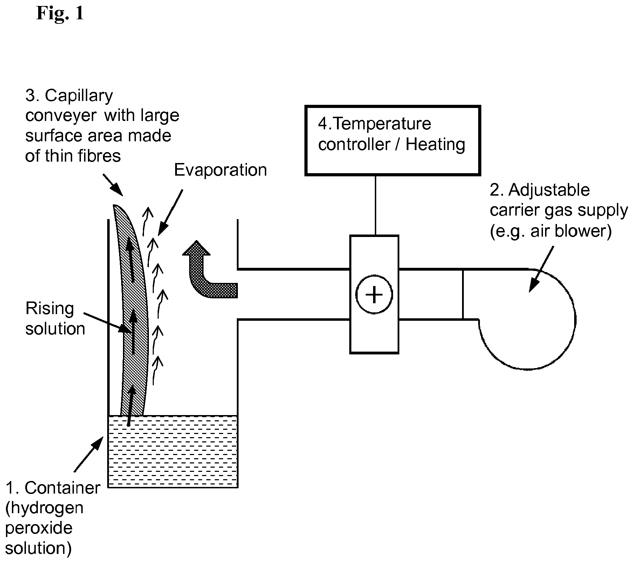

According to one embodiment of the invention (see Fig. 1), the present device for generating vaporous hydrogen peroxide (H₂O₂), includes a container 1 for holding a liquid solution of hydrogen peroxide (H₂O₂), the container 1 having an opening, a temperature controlled carrier gas supply 2 being an electrical gas blower 2, most suitably a hot air blower, and a flat fibrous capillary conveyer 3 capable of drawing up liquid, the capillary conveyer 3 preferably being made of glass fiber.

The device also includes one or more of means 4 for adjusting the temperature in the carrier gas supply 2 (see Fig. 1), means 5 for adjusting the flow in the carrier gas supply 2, and means 6 for holding the capillary conveyer 3 at the opening of the container 1.

The container 1 is typically shaped like a cylinder or a funnel (that is thinner at the open end), with one opening that fits one end of the capillary conveyer 3, and typically a separate opening suitable for use as a carrier gas inlet.

The carrier gas supply 2 is typically connected to the container 1, so that gas can be blown into the container 1 at a point below its opening, preferably at a point that is located 1/3 - 1/8 of the distance from the opening of the container 1 to the bottom of the container 1.

The carrier gas is selected from any non-reactive (or inert) gas or gas mixture, such as nitrogen, argon or air, or a mixture of any of these. Typically, nitrogen or air is used.

The capillary conveyer 3 is preferably essentially flat, has a width smaller than the diameter of the opening of the container 1, for example a width of 3/4 - 7/8 of the diameter of the opening of the container 1, and has a length extending from the opening of the container 1 to the bottom of the container 1, whereby one end of the capillary conveyer 3 can be immersed into the liquid, while the other end is held at the opening of the container 1. Most suitably, it consists of a large number of thin non-absorbing solid fibres (e.g glass fibres) forming totally a large area for evaporation.

The process for generating vaporous hydrogen peroxide (H₂O₂) includes the steps of providing a container 1 holding a hydrogen peroxide solution, impregnating the capillary conveyer 3 (such as a glass fiber fabric) with hydrogen peroxide, and blowing carrier gas onto it, whereby the hydrogen peroxide is vaporized.

Thus, the method includes vaporizing a liquid solution of hydrogen peroxide to form hydrogen peroxide vapor. The liquid solution to be evaporated is typically an aqueous solution, preferably having a hydrogen peroxide concentration of 5-50 vol-%. more preferably 30-35 vol-%.

The temperature of the carrier gas blowing onto the capillary conveyer 3 from the above mentioned carrier gas supply 2 is adjusted, whereby the the flow rate can also be adjusted.

The flow rate of the carrier gas is typically adjusted to a suitable level, based on parameters such as the size of the container 1, the size of the opening of the container 1, and the surface area of the capillary conveyer 3.

According to an embodiment, the flow rate is adjusted to a level of 120 - 200 l/min, although flow rates up to 480 1/min can be easily achieved when necessary.

The temperature of the supplied carrier gas is maintained at <100 °C, preferably 40 ― 90 °C. The pressure is not adjusted, whereby it remains at around ambient pressure.

The humidity of the air in the space to be decontaminated needs to be below the dew point of the mixture of water and hydrogen peroxide vapour, often requiring drying of the air in the space.

The space to be decontaminated using a single device of the embodiments of the present invention is typically < 50 m³, although larger spaces can be treated by simultaneously using more than one device.

An effective concentration of H₂O₂ vapor, of about 300 to about 500 ppm, typically between 350 and 400 ppm, in the above mentioned room size of < 50 m³, is typically achieved within 4h, although 3.5h is generally sufficient.

According to the above embodiments, hydrogen peroxide is generated using a gentle and efficient evaporation method. The hydrogen peroxide solution is drawn up by the capillary conveyer, having a large evaporation area, and is evaporated with the aid of the heated flow of carrier gas. While the H₂O₂ is evaporated, more solution can be drawn up by the capillary conveyer, whereby the method is continuous, and gives a steady and high yield of gaseous hydrogen peroxide.

The invention is useful particularly for disinfecting purposes (i.e. killing microbes) in, e.g., hospitals and the food industry.

The hydrogen peroxide vapour generated according to the present invention should not be allowed to condensate, as this could result in corrosion. Therefore, the atmosphere is kept sufficiently dry during the entire disinfecting procedure, and a suitable temperature is maintained.

Preferably the hydrogen peroxide concentration is 5-50 % by weight, more preferably 30-35% by weight aqueous hydrogen peroxide. At this level, condensation of hydrogen peroxide is limited, while disinfection in a short period of time is achieved.

Further, the process conditions arc adjusted based on the target to be decontaminated, such as its volume or surface area.

In the decontamination process, the space to be decontaminated is closed, possibly dried using a suitable dryer selected based on the space to be decontaminated, and filled with H₂O₂ vapour. A sufficient amount of hydrogen peroxide is constantly generated to maintain a steady concentration of gas in the space for a sufficient amount of time (e.g. 1 - 2 h). After the disinfection is considered complete, ventilation of the space is provided, and preferably equipped with filtering of the exhaust air.

The invention facilitates decontamination of a wide range of microbes in e.g. hospitals, food industry, transport vehicles and pods and in the security and defense sectors.

The most important application area of the invention, in one embodiment, is its use in microbe decontamination in infectious disease control.

The following non-limiting example is intended merely to illustrate the advantages obtained with the embodiments of the present invention.

### EXAMPLE

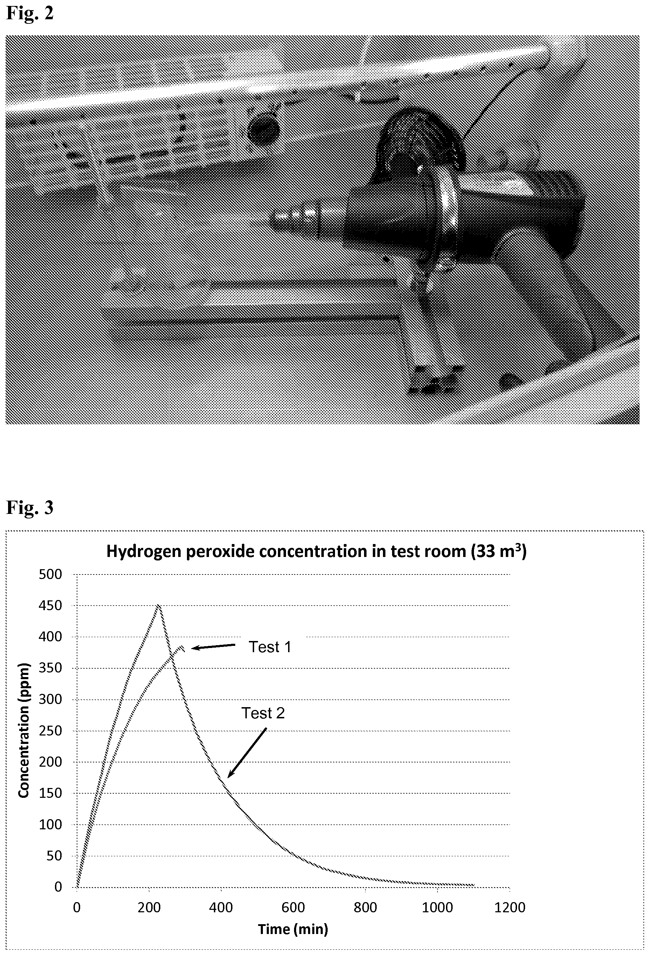

A decontamination was carried out in a test room of 33 m³, by generating hydrogen peroxide from an aqueous 35 vol-% H₂O₂ solution using the device shown in Figure 2.

In about 200 minutes, a H₂O₂ vapor concentration of 400 - 450 ppm (the results of two identical measurements) was achieved, while only using up about 40 ml of the hydrogen peroxide solution.

The results of the decontamination are shown in Figure 3.

### Industrial Applicability

The present device and the present process can be used in generating hydrogen peroxide to decontaminate or disinfect spaces, such as hospital rooms, facilities of the food industry, or vchiclcs, such as ambulances or army vchiclcs, and generally for replacement of conventional complex vapour generators that utilize high pressures and sometimes high temperatures.

### Reference signs

- 1: Container for holding a liquid
- 2: Temperature controlled carrier gas supply
- 3: Capillary conveyer
- 4: Means for adjusting the temperature
- 5: Means for adjusting the flow of carrier gas
- 6: Holding means

### Citation List

JPH09272515 A
US2011176959 A1
US 20120277662
US 5792435
WO9715333 A1

## Claims

1. A device suitable for generating vaporous hydrogen peroxide (H₂O₂), including a container (1) for holding a liquid solution of hydrogen peroxide (H₂O₂), the container (1) having an opening, which further includes a temperature controlled carrier gas supply (2) in the form of an electrical gas blower, and a fibrous capillary conveyer (3) capable of drawing up liquid, **characterised in that** it includes means (4) for adjusting the temperature in the carrier gas supply (2).

2. The device of claim 1,
including means (5) for adjusting the flow of gas in the carrier gas supply (2).

3. The device of claim 1 or 2, wherein the carrier gas supply (2) is connected to the container (1), so that carrier gas can be blown into the container (1) at a point below its opening, preferably at a point that is located 1/3 - 1/8 of the distance from the opening of the container (1) to the bottom of the container (1).

4. The device of any preceding claim, wherein the capillary conveyer (3) is made of glass fiber.

5. The device of any preceding claim, wherein the capillary conveyer (3) is essentially flat, has a width smaller than the diameter of the opening of the container (1), and has a length extending from the opening of the container (1) to the bottom of the container (1), whereby one end of the capillary conveyer (3) can be immersed into the liquid, while the other end is held at the opening of the container (1).

6. The device of any preceding claim, including means (6) for holding the capillary conveyer (3) at the opening of the container (1).

7. A process for generating vaporous hydrogen peroxide (H₂O₂), which includes providing a container (1) holding a hydrogen peroxide solution, drawing the hydrogen peroxide into a capillary conveyer (3), whereby the capillary conveyer (3) is essentially impregnated with the solution, and blowing carrier gas onto it in order to vaporize the hydrogen peroxide, the process being **characterised in that** the temperature of the carrier gas blown onto the capillary conveyer (3) is adjusted to a temperature of < 100 °C.

8. The process of Claim 7, wherein the capillary conveyer (3) is selected from flat glass fiber capillary conveyers (3).

9. The process of Claim 7 or 8, wherein the carrier gas is selected from air, nitrogen and argon, or any mixture of these.

10. The process of any of claims 7 to 9, wherein the temperature of the carrier gas blown onto the capillary conveyer (3) is adjusted to a temperature 40-90 °C.

11. The process of any of claims 7 to 10, wherein the flow rate of the gas blown onto the capillary conveyer (3) is adjusted to a level of 120-200 l/min.

12. Use of the device of any of claims 1 to 6 or of the process of any of claims 7 to 11 for disinfecting purposes in hospitals, in the food industry, or in transport vehicles or pods, defense or security sectors.

## Patentansprüche

1. Vorrichtung, die zur Erzeugung von dampfförmigem Wasserstoffperoxid (H₂O₂) geeignet ist, einschließlich einem Behälter (1) zur Aufnahme einer flüssigen Wasserstoffperoxidlösung (H₂O₂), wobei der Behälter (1) eine Öffnung aufweist, die weiter eine temperaturgesteuerte Trägergaszufuhr (2) in Form eines elektrischen Gasgebläses und einen faserigen Kapillarförderer (3), der Flüssigkeit aufnehmen kann, einschließt, **dadurch gekennzeichnet, dass** sie Mittel (4) zum Einstellen der Temperatur in der Trägergaszufuhr (2) einschließt.

2. Vorrichtung nach Anspruch 1,
einschließlich Mittel (5) zum Einstellen des Gasflusses in der Trägergaszufuhr (2).

3. Vorrichtung nach Anspruch 1 oder 2, wobei die Trägergaszufuhr (2) mit dem Behälter (1) verbunden ist, so dass Trägergas in den Behälter (1) an einer Stelle unterhalb seiner Öffnung eingeblasen werden kann, bevorzugt an einer Stelle, die 1/3 - 1/8 des Abstandes von der Öffnung des Behälters (1) zum Boden des Behälters (1) beträgt.

4. Vorrichtung nach einem der vorstehenden Ansprüche, wobei der Kapillarförderer (3) aus Glasfaser hergestellt ist.

5. Vorrichtung nach einem der vorstehenden Ansprüche, wobei der Kapillarförderer (3) im Wesentlichen flach ist, eine Breite aufweist, die kleiner als der Durchmesser der Öffnung des Behälters (1) ist, und eine Länge aufweist, die sich von der Öffnung des Behälters (1) bis zum Boden des Behälters (1) erstreckt, wodurch ein Ende des Kapillarförderers (3) in die Flüssigkeit eingetaucht werden kann, während das andere Ende an der Öffnung des Behälters (1) gehalten wird.

6. Vorrichtung nach einem der vorstehenden Ansprüche, einschließlich Mittel (6) zum Halten des Kapillarförderers (3) an der Öffnung des Behälters (1).

7. Verfahren zur Erzeugung von dampfförmigem Wasserstoffperoxid (H₂O₂), das die Bereitstellung eines Behälters (1), der eine Wasserstoffperoxidlösung enthält, das Einsaugen des Wasserstoffperoxids in einen Kapillarförderer (3), wodurch der Kapillarförderer (3) im Wesentlichen mit der Lösung imprägniert wird, und das Aufblasen von Trägergas auf diesen umfasst, um das Wasserstoffperoxid zu verdampfen, wobei das Verfahren **dadurch gekennzeichnet ist, dass** die Temperatur des auf den Kapillarförderer (3) geblasenen Trägergases auf eine Temperatur von < 100 °C eingestellt wird.

8. Verfahren nach Anspruch 7, wobei der Kapillarförderer (3) aus flachen Glasfaserkapillarförderern (3) ausgewählt ist.

9. Verfahren nach Anspruch 7 oder 8, wobei das Trägergas ausgewählt ist aus Luft, Stickstoff und Argon oder einem beliebigen Gemisch davon.

10. Verfahren nach einem der Ansprüche 7 bis 9, wobei die Temperatur des auf den Kapillarförderer (3) geblasenen Trägergases auf eine Temperatur von 40-90 °C eingestellt wird.

11. Verfahren nach einem der Ansprüche 7 bis 10, wobei die Strömungsgeschwindigkeit des auf den Kapillarförderer (3) geblasenen Gases auf einen Wert von 120-200 l/min eingestellt wird.

12. Verwendung der Vorrichtung nach einem der Ansprüche 1 bis 6 oder des Verfahrens nach einem der Ansprüche 7 bis 11 zu Desinfektionszwecken in Krankenhäusern, in der Lebensmittelindustrie oder in Transportfahrzeugen oder - kapseln, im Verteidigungs- oder Sicherheitsbereich.

## Revendications

1. Dispositif approprié pour générer du peroxyde d'hydrogène (H₂O₂)vaporeux, incluant un récipient (1) pour contenir une solution liquide de peroxyde d'hydrogène (H₂O₂), le récipient (1) présentant une ouverture, qui inclut en outre une alimentation en gaz vecteur (2) à température régulée sous la forme d'une soufflante électrique de gaz, et un transporteur capillaire fibreux (3) capable d'aspirer un liquide, caractérisé en qu'il inclut un moyen (4) pour ajuster la température dans l'alimentation en gaz vecteur (2).

2. Dispositif selon la revendication 1,
incluant un moyen (5) pour ajuster le débit de gaz dans l'alimentation en gaz vecteur (2).

3. Dispositif selon la revendication 1 ou 2, dans lequel l'alimentation en gaz vecteur (2) est raccordée au récipient (1), de sorte qu'un gaz vecteur peut être soufflé dans le récipient (1) à un endroit en dessous de son ouverture, de préférence à un endroit qui est situé à 1/3-1/8 de la distance allant de l'ouverture du récipient (1) jusqu'au fond du récipient (1).

4. Dispositif selon une quelconque revendication précédente, dans lequel le transporteur capillaire (3) est fait de fibres de verre.

5. Dispositif selon une quelconque revendication précédente, dans lequel le transporteur capillaire (3) est essentiellement plat, présente une largeur inférieure au diamètre de l'ouverture du récipient (1), et présente une longueur s'étendant de l'ouverture du récipient (1) jusqu'au fond du récipient (1), selon lequel une extrémité du transporteur capillaire (3) peut être immergée dans le liquide, tandis que l'autre extrémité est maintenue au niveau de l'ouverture du récipient (1).

6. Dispositif selon une quelconque revendication précédente, incluant un moyen (6) pour maintenir le transporteur capillaire (3) au niveau de l'ouverture du récipient (1).

7. Procédé de génération de peroxyde d'hydrogène (H₂O₂)vaporeux, qui inclut
la fourniture d'un récipient (1) contenant une solution de peroxyde d'hydrogène, l'aspiration du peroxyde d'hydrogène dans un transporteur capillaire (3), selon lequel le transporteur capillaire (3) est essentiellement imprégné avec la solution, et le soufflage d'un gaz vecteur sur celui-ci afin de vaporiser le peroxyde d'hydrogène, le procédé étant **caractérisé en ce que** la température du gaz vecteur soufflé sur le transporteur capillaire (3) est ajustée à une température < 100 °C.

8. Procédé selon la revendication 7, dans lequel le transporteur capillaire (3) est sélectionné parmi les transporteurs capillaires (3) à fibres de verre plates.

9. Procédé selon la revendication 7 ou 8, dans lequel le gaz vecteur est sélectionné parmi l'air, l'azote et l'argon, ou tout mélange de ceux-ci.

10. Procédé selon l'une quelconque des revendications 7 à 9, dans lequel la température du gaz vecteur soufflé sur le transporteur capillaire (3) est ajustée à une température de 40-90 °C.

11. Procédé selon l'une quelconque des revendications 7 à 10, dans lequel le débit du gaz soufflé sur le transporteur capillaire (3) est ajusté à un niveau de 120-200 l/min.

12. Utilisation du dispositif selon l'une quelconque des revendications 1 à 6 ou du procédé selon l'une quelconque des revendications 7 à 11 à des fins de désinfection dans les hôpitaux, dans l'industrie alimentaire, ou dans les véhicules ou nacelles de transport, les secteurs de la défense ou de la sécurité.
